# EUROPEAN PATENT SPECIFICATION

(11) **EP 3 750 862 B1**
(45) Date of publication and mention of the grant of the patent: **28.09.2022**
(21) Application number: 19179358.7
(22) Date of filing: 11.06.2019
(51) Int. Cl.: C05F 17/50, C05F 17/957, C05F 17/971, C12M 1/107, C12M 1/06

(54) **ANAEROBIC BIOGAS PLANT AND DIGESTION METHOD**
ANAEROBE BIOGASANLAGE UND GÄRUNGSVERFAHREN
INSTALLATION DE BIOGAZ ANAÉROBIE ET PROCÉDÉ DE DIGESTION

(43) Date of publication of application: 16.12.2020
(73) Proprietor: Hitachi Zosen Inova AG, 8005 Zürich (CH)
(72) Inventor: SCHATZ, Adrian, 3172 Niederwangen (CH); LEISNER, Rene, 78467 Konstanz (DE)
(74) Representative: Schaad, Balass, Menzl & Partner AG

(56) References cited:
- EP-A1- 1 930 404
- US-A1- 2015 048 008
- US-A1- 2016 024 449
- US-A1- 2016 298 067
- US-A1- 2017 029 309

## Description

The present invention relates to an anaerobic biogas plant according to Claim 1 and to a digestion method according to claim 7.

In general, the technical field of the present invention is the anaerobic digestion - sometimes also referred to as "fermentation" - of biomass, i.e. organic matter present in various types of wastes (sludge, agro-industrial wastes, energy crops), in an anaerobic biogas plant. The technology of anaerobic digestion is a biochemical process accomplished by the combined action of a group of several types of microorganisms, which metabolize the organic materials into a gaseous mixture consisting of mainly methane and carbon dioxide (biogas) under anaerobic conditions.

Anaerobic digestion generally consists of four steps: hydrolysis, acidogenesis, acetogenesis, and methanogenesis. High molecular weight substrates are first hydrolyzed by fermentative bacteria into low molecular weight and water soluble organic intermediate products such as glucose, fatty acids and amino acids. During acidogenesis, volatile fatty acids (VFAs) are derived, along with the generation of byproducts including NH₃, CO₂ and H₂S. The VFAs are then converted into acetate, more CO₂, H₂, etc., via acetogenesis. It is the acetate that methanogens act on to produce biomethane during the final methanogenesis process. Among the four steps, hydrolysis is the slowest and is therefore the rate-determining step.

The biogas is generally upgraded and then either sent to a natural gas network or used directly for the production of electricity, heat and/or transportation fuel. The final digestate remaining after the digestion process is used as soil conditioner and fertilizer.

To provide the required anaerobic conditions, vertical or horizontal digestion tanks are generally used. One example of a horizontal digester that is operated in plug-flow mode and is suitable for anaerobic digestion of biogenic wastes is described in European patent EP 0 621 336. The digester is an elongated, horizontal tank having an inlet at one end and an outlet at the opposite end. Chopped biogenic wastes are supplied through the inlet and are inoculated by means of digested material and press water from the post-processing after digestion. By this, the substrate to be digested is enriched with methanobacteria. In the digester, the biogenic wastes are now decomposed under controlled mixing forming biogas and are subsequently, after exiting through the outlet, supplied to post-processing incl. dewatering and aerobic rotting. Further anaerobic biogas plants and digestion methods are disclosed in EP 1 930 404 A1, US 2017/029309 A1, US 2016/298067 A1, US 2015/048008 A1 and US 2016/024449 A1.

The worldwide demand for anaerobic biogas plants with larger capacities led to the construction of increasingly large digesters. Nowadays, horizontal digester tanks of a total length of 50 meters and having a diameter of more than 10 meters can be realized. This increase in size came along with technical difficulties associated with the transport of the digestion substrate through the digester, as well as with the control of the digestion process. Specific issues concern the provision of a certain homogeneity of the digestion substrate and the prevention of the sedimentation of heavy solids, such as in particular sand and stones, at the bottom of the digester tanks. Sedimentation prevention ensures continuous discharge of the digested substrate. Even when operating the digester in plug-flow mode, the heavier goods tend to sink because of the low flow velocity and thus cannot be discharged. As a result, the increase in size of the digester often came at the expense of a decreased digestion quality and longer retention time resp. the passing time of the biogenic wastes through the digester from the inlet to the outlet.

For efficient digestion a balanced microbial consortium of hydrolysing bacteria, acidogenic bacteria, acetogens and methanogens are required in the system. It was found that the addition of some digested substrate to the mass of fresh digestion substrate introduced into the digester leads to a reduction of the overall retention time (i.e. dwell time of the digestion substrate). In accordance therewith, EP 1 930 404 discloses the use of some digested substrate from the digester itself for inoculation of the fresh biomass introduced into the digester ("self-inoculation"). Some of the digested biomass retrieved after a total retention time is therefore returned to the inlet area of the digester. By operating a plurality of fermenters in parallel to each other, the overall biomass throughput of a biogas plant could be increased. The primary goal of the method of EP'404 is to maximize the amount of collectable biogas produced during the digestion and to make sure that all germs are killed efficiently.

Other methods, such as described for instance in GB 720,018 do not rely on classic self-inoculation. GB'018 discloses an anaerobic digestion method for biomass, wherein several fermenter chambers are used, through which the digestion substrate is fed in a predetermined order. The digester liquor formed while digestion in the fermenter chambers is collected and supplied to a separate inoculation fermenter, such that bacteria may be reused in the inoculation fermenter to produce inoculation substrate.

While both of EP'404 and GB'018 present ways to improve the digestion process, they do not specifically focus on increasing the overall throughput of biomass through the biogas plant. This has been a primary objective of the biogas plant and conversion method disclosed in European Patent EP 2 948 537, which is owned by the Applicant of the present application. The method of EP'537 involves the return of some substrate from an inoculum fermenter chamber operated in the plug-flow mode back to the inoculum fermenter chamber by means of a return path. In addition, the remaining, not returned substrate is supplied to at least one further fast fermenter chamber - also operated in the plug-flow mode - by means of a fast inoculation path and is mixed there with additional fresh fermentation substrate. This means the at least one fast fermenter chamber does not require a self-inoculation, which allows for achieving a significantly shorter dwell time in the fast fermenter chamber and, as a result, for increasing the overall throughput of raw fermentation substrate of the biogas plant compared to a biogas plant having the same number/volume of fermenter chambers operated in parallel.

Not disregarding the benefits provided by the biogas plant and conversion method described in EP'537, there is still room for improvement with respect to maximize biogas production and to decrease the construction and maintenance costs of the biogas plant.

The problem solved by the present invention is thus to provide an anaerobic biogas plant and digestion method that not only maximizes the total throughput of the digestion substrate but also allows a reduction of construction and maintenance costs.

This problem is solved by the anaerobic biogas plant according to Claim 1 and the digestion method of claim 7.

Preferred embodiments are subject to the dependent claims. In line with the present invention, an anaerobic biogas plant is provided that comprises a digester for the digestion of fresh organic digestion substrate. It goes without saying that the digester provides an environment that allows for the digestion of organic material. In general, this means that the digester contains microorganisms that are capable of digesting the digestion substrate.

In accordance with the present invention, the digester has an entry section with a main inlet at a first end, a rear section at a second opposite end, and a middle section arranged between the entry section and the rear section. The digester further includes a stirring unit comprising a shaft extending in the direction from the first end to the second end and having at least one agitating arm for mixing the digestion substrate within the digester.

A key aspect of the present invention is that a major fraction of digestion substrate is removed from the digester after a shortened retention time t. Said shortened retention time t is set with the focus on maximum biogas production. To this end, the middle section of the digester includes a main outlet for removal of a major fraction of digestion substrate after a shortened retention time t. The term "retention time" is typically expressed in hours or days and refers to the time required to achieve a degradation degree of the digestion substrate in view of either maximum biogas production or production of an inoculum substrate.

On the other hand, only a minor fraction of digestion substrate is kept within the digester for inoculum retention time T that is longer than the shortened retention time t. Said inoculum retention time T is set to provide the digestion substrate with inoculation capacity. As such, in line with the present invention, the minor fraction of digestion substrate is removed from the digester after the inoculum retention time T and directly re-introduced into the digester, where it is mixed with fresh digestion substrate and acts as inoculation substrate. For this purpose, the rear section of the digester includes an additional outlet and a separate return line. The additional outlet serves for removal of the minor fraction of digestion substrate after the inoculum retention time T. The separate return line leads from the additional outlet back to the entry section of the digester and is provided for directly returning the minor fraction removed from the rear section back into the entry section of the digester. The term "separate" thereby indicates that the return line is exclusively intended for the return of the minor fraction of digestion substrate and does not serve other purposes.

The microbial consortium in the anaerobic digestion process consists of several different microorganism groups which grow syntrophically or antagonistically, resulting in different responses to environmental changes. Therefore, when the activity of one of the microorganism groups is repressed, the growth of other microorganism groups is affected, the microbial population is changed, which may lower process efficiency or even cause a process failure. Thanks to the addition of the inoculation substrate, rapid onset of a balanced microbial population is accomplished, with the effect that the digestion of the digestion substrate within the digester is accelerated, meaning that less retention time (or "dwell time" or "digestion time") is required to produce a certain amount of biogas compared to digesters not using an inoculation substrate. In consequence, the anaerobic biogas plant of the present invention allows for increasing the overall throughput of fresh digestion substrate and thus maximizing the amount of biogas produced within the digester.

Also, owing to this specific design of the inventive digester with an additional outlet and a separate return line, the anaerobic biogas plant of the present invention allows for the inoculation of the fresh digestion substrate as well as the preparation of an inoculation substrate within one and the same digester and nevertheless securing the incessant operation of the anaerobic digestion. An additional inoculation digester is therefore not required. The major advantages of the inventive anaerobic biogas plant therefore lie in the shortened retention time and in its simplicity in construction and operation of the biogas plant.

Throughout this application, the term "separate return line" refers to a pipe that is exclusively provided for the return of substrate from the rear section after a prolonged inoculum retention time into the entry section of the digester. The term "prolonged retention time" refers to the longer "inoculum retention time T" compared to the "shortened retention time t" of the substrate removed through the additional outlet of the middle section. The digestion degree of the substrate removed from the rear section is thereby such that it can be used as inoculation substrate that accelerates the digestion process. One key process indicator for the quality or suitability of the digestion substrate removed from the rear section of the digester to be used as inoculation substrate is the concentration of volatile fatty acids (VFAs) in the digestion substrate, which has a direct influence on the pH value thereof. Common limits for inoculation substrate are e.g. 500 mg/l of acetic acid, 250 mg/l of propionic acid and 100 mg/l of butanoic acid.

The addition of inoculation substrate to fresh digestion substrate can occur before or at the time of entering the digester. More specifically, the inoculation substrate may be either be re-introduced into the entry section via the main inlet or via an additional separate inlet. For instance, a delivery pipe may be provided for the delivery of fresh digestion substrate into the digester. Said delivery pipe may include a T-piece that connects the return line with the delivery pipe, such that the inoculation substrate is added to the fresh digestion substrate before being introduced into the digester.

It is known that the temperature is one of the most important operating parameters in the anaerobic digestion because temperature directly affects the activity of various enzymes and related co-enzymes, sludge quality, hydrolysis rates, and subsequent biogas production. Anaerobic digestion microorganisms can typically function at three different temperature ranges: psychrophilic (optimally below 20°C), mesophilic (optimally at 35-40°C), and thermophilic (optimally at 50-65°C). From the standpoint of biochemical reactions, higher anaerobic digestion performance can be achieved when temperature increases within bounded limits. A too low digester temperature may impede an optimal catalytic efficiency of the enzymes, while a too high digester temperature may denature sensitive enzymes and subsequently lead to process failure. Generally, it was found that, compared with mesophilic temperature condition, thermophilic operation exhibited better performance in accelerating endergonic metabolic reactions, promoting biogas production and relieving ammonia accumulation. On the other hand, thermophilic anaerobic digestion operation has shown to be unfavorable to exergonic metabolic reactions, more vulnerable to changes in environmental conditions and might not be applicable to special substrates. For the biogas plant of the present invention, mesophilic anaerobic digestion is preferably applied for digesting rumens of animals and biowaste feedstocks from industrial and farm activities, while thermophilic anaerobic digestion is preferably applied for sanitation of pathogen-bearing digestion substrate.

In a preferred embodiment the digester is an elongated, horizontal tank, preferably with an essentially constant diameter. This facilitates the construction of the digester as well as the transport of digestion material therethrough.

The shaft of the stirring unit extends from the entry section through the middle section into the rear section of the digester. In general, the sections of the digester are not physically separated from one another, e.g. by means of separation walls. Therefore, the construction of the stirring unit and also of the digester is facilitated.

Apart from mixing the digestion substrate in order to achieve a certain homogeneity, the stirring unit generally also helps to prevent the sedimentation of heavy solids at the bottom of the digester. Preferably, the shaft of the stirring unit has a multitude of agitating arms, which are provided with suitable blades at their free ends, i.e. at their ends remote from the shaft. The blades are intended to move heavy goods from the bottom towards the top again, such that during the subsequent sinking they can be moved in the direction towards the outlet of the digester.

In order to facilitate continuous transport of the digestion substrate through the digester, the digester is preferably operated in the plug-flow mode. In particular when using a horizontal digester, the use of a plug-flow mode of operation helps to reduce the required retention time of fresh digestion substrate in the digester to achieve a high biogas production and thus results in an increased throughput of the anaerobic biogas plant (compared to an identical digester not operated in plug-flow mode). In addition, the use of a plug-flow operation mode enables different process conditions to be present at the entry section, the middle section and the rear section. This multistage configuration is designed to produce digested substrate with a minimal odor level, better dewatering properties and low content in pathogens.

Notably, for the digestion of essentially liquid materials, i.e. so-called "low solid content feedstocks" (e.g. secondary wastewater treatment, wastewater from food industry, hydraulic flush manure systems), a plug-flow mode can generally not be applied. Therefore, if the digestion chamber is operated in plug-flow mode, the digestion material is preferably a mixture of liquid and solid materials, in particular having a solid content of at least 10%. In this case, the mixing unit is preferably provided with slowly rotating impellers that can aid the horizontal movement of the digestion substrate mixture and also serves for mixing, degassing and suspension of the heavier particles.

Preferably, the separate return line exclusively connects the rear section with the entry section of the digester. This means that there is no other functional unit of the anaerobic biogas plant, in particular no additional digester, connected to the return line. As such, the removed minor fraction of digestion material can be directly returned, i.e. re-circulated, into the digester.

Since the anaerobic biogas plant of the present invention includes a digester that produces both, digested substrate (after a shortened retention time t) and inoculation substrate (after an inoculum retention time T, with T>t), no separate inoculum digester is required. Therefore, the anaerobic biogas plant of the present invention preferably includes only one or more digester (s) that are identical with respect to their construction and function. This means that preferably, the digester(s) all include at least a middle section with a main outlet, a rear section with an additional outlet and a separate return line as described above.

The present invention also relates to a digestion method for use in an anaerobic biogas plant as described herein. Thus, the inventive digestion method is for use in an anaerobic biogas plant having at least one digester of the type described above, i.e. a digester having an entry section with a main inlet at a first end, a rear section at a second opposite end, a middle section arranged between the entry section, a main outlet from the middle section, an additional outlet from the rear section, a separate return line leading from the additional outlet directly back into the entry section of the digester, and a stirring unit with a shaft extending in the direction from the first to the second end and having at least one agitating arm for mixing a digestion substrate within the digester. The method comprising the steps of:
a) supplying fresh digestion substrate through the main inlet into the digester;
b) removing a minor fraction of digestion substrate via the additional outlet from the rear section of the digester after an inoculum retention time T,
c) returning the minor fraction of digestion substrate via the return line directly back into the entry section of the digester to act as inoculation substrate,
d) mixing the inoculation substrate with fresh digestion substrate to provide a combined digestion substrate, and
e) removing a major fraction of combined digestion substrate via the main outlet from the middle section of the digester after a shortened retention time t.

In line with the method of the present invention, the major fraction of combined digestion substrate is larger than the minor fraction of digestion substrate (acting as inoculation substrate), and the shortened retention time t is shorter than the inoculum retention time T.

In a preferred embodiment, the minor fraction of digestion substrate removed from the rear section is directly returned back into the entry section of the digester without further processing. This means that the same material that is retrieved from the digester is also re-introduced into the latter. This is in contrast to e.g. the method described in GB'018, in which digested liquor is separated from the digested substrate and used as inoculum in an inoculum digester.

Preferably, the minor fraction of digestion substrate removed in step d) is less than 50%, preferably less than 40%, more preferably about 30% or less, of the amount of fresh digestion substrate fed into the digester. It was found that an amount of 20% to 30% of inoculation substrate in relation to the overall input of fresh digestion substrate into the digester is sufficient for inoculation purposes, i.e. to drive the digestion process.

It was found that the retention time required to produce a certain amount of biogas can be shortened by the addition of the inoculation substrate, down to a third of the standard retention time or even less. The shorter the retention time, the higher the overall throughput of the anaerobic biogas plant. Nevertheless, if the retention time is set too low it leads to fatty acid accumulation, which can cause bacteria inhibition and even process failure. Therefore, the shortened retention time t is preferably set to the time at which 80-95% of the (theoretical) maximum biogas output obtainable from the digestion of the digestion substrate is achieved. The maximum biogas output, i.e. the amount of biogas or methane that can be produced per unit mass of the digestion substrate within a given period under a given temperature, can be calculated by methods known in the art. One good method of evaluation was e.g. developed by Prof. Dohanyos and Doc. Zábranská from the Institute of Chemical Technology (ICT) (Straka et al. 2003).

More specifically, is preferred that the shortened retention time t is at least 2 days, more preferably at least 4 days and 10 days at most, most preferably between 5 and 8 days.

The production of an inoculum substrate, on the other hand, requires more time. For the biogas plant of the present invention the inoculum retention time T of the minor fraction of digestion substrate is preferably at least 10 days, more preferably at least 14 days, most preferably about 21 days. An inoculum retention time of about 21 days has been shown to result in a substrate with a degree of digestion that makes it particularly efficient as inoculation substrate.

With regard to the shortened retention time t of the combined digestion substrate it is preferred that said shortened retention time t is less than 50%, preferably less than 40%, more preferably about a third of the inoculum retention time T, i.e. of the time after which the minor fraction of digestion substrate is removed through the outlet at the rear section to be used as inoculation substrate.

In a preferred embodiment, the recirculation - meaning the removal and return of the minor fraction of digestion substrate from and back into the digester - occurs in a continuous fashion or in regular time intervals. Thus, the digester may be operated in a continuous or batch mode of operation. This means that it can be operated in a continuous mode or loaded in batches and emptied upon completion of the waste degradation up to a degree (with the exception of the minor fraction).

Alternatively, it is preferred that the removal and return of the minor fraction of digestion substrate from and back into the digester occurs in dependence of at least one condition parameter measured within the digester, said at least one condition parameter is selected from the group consisting of pH, volume of substrate, temperature, content of volatile fatty acids, free ammonia concentration and partial gas pressure.

If the fresh digestion substrate has a high solid content it is preferably mechanically pretreated, e.g. by milling, to accomplish a particle size reduction to thereby increase the surface area and thus an increased substrate availability for the microorganisms.

It was found that pH is a crucial parameter in dynamic detection and regulation of the anaerobic digestion process due to the fact that operation stability and activities of acidogenic and methanogenic bacteria are significantly affected by changes in pH. For optimal operation, the pH within the digester is preferably monitored and kept within a range of 6-8. If necessary, acidic (e.g. HCl) or basic (e.g. NaHCO₃) solutions can be introduced into the digester.

The present invention is further described in connection with the enclosed drawings, which show purely schematically:

### Figures

- Fig. 1: a schematic view of a biogas plant according to the state in the art without a separate inoculation digester, but having only one single digester which is supplied with substrate;
- Fig. 2: a schematic view of an alternative biogas plant according to the state in the art, the biogas plant having a separate inoculation digester and a fast digester, which are coupled to each other and are supplied with substrate; and
- Fig. 3: a schematic view of an anaerobic biogas plant having a single digester carrying out both functions of an inoculation digester and a fast digester in accordance with the present invention.

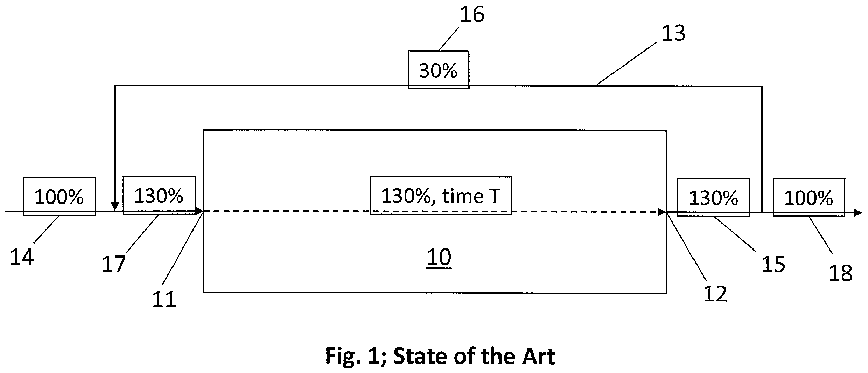

Fig. 1 shows a schematic representation of the digestion process in an anaerobic biogas plant according to the state of the art. Said biogas plant includes a single digester 10 with an inlet 11 at one end, an outlet 12 at an opposite end and a return path 13 connecting the outlet 12 with the inlet 11. Fresh digestion substrate 14 is introduced into the digester 10 and digested therein for a total standard retention time T, before being removed as (essentially fully) digested substrate 15. The term (essentially fully) digested substrate thereby refers to a substrate that has passed the four main stages of digestion, i.e. hydrolysis, acidogenesis, acetogenesis, and methanogenesis. Therefore, pH, methane yield and the concentration of volatile fatty acids (VFAs) in the digestion substrate are indicators of how far the digestion has progressed. A minor fraction 16 of the digested substrate 15 removed from the digester 10 is returned through the return path 13 back into the digester 10 to act as "self-inoculation substrate" that accelerates the digestion process.

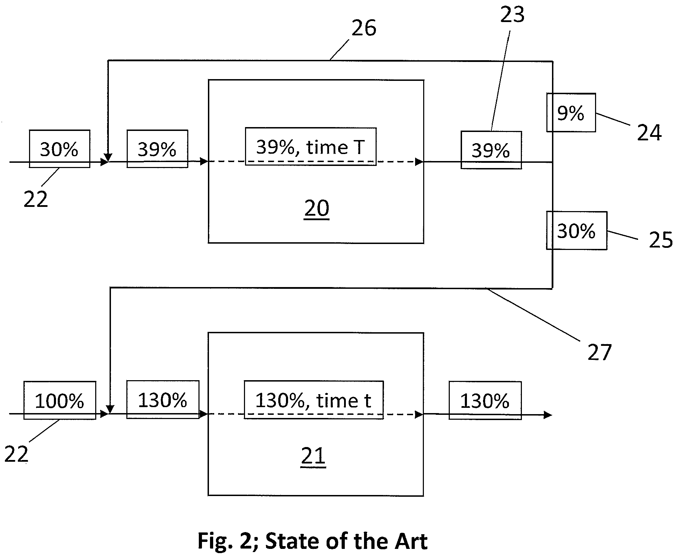

The biogas plant shown in Fig. 2 is operated in accordance with the method described in EP 2 948 537. In this biogas plant, an inoculum digester 20 is coupled with at least one further fast digester 21. Fresh digestion substrate 22 is introduced into the inoculum digester 20 and digested for a time T before leaving the inoculum digester 20 as inoculating substrate 23. A minor fraction 24 of the inoculating substrate 23 from the inoculum digester 20 is returned to the inoculum digester 20 by means of a return path 26 to act as self-inoculate and a major fraction 25 of the inoculating substrate 23 is supplied to the fast digester 21 by means of a fast inoculation path 27. The major fraction 25 of inoculating substrate 23 is mixed in the fast digester 21 with additional fresh digestion substrate 22. Because the inoculum digester 20 produces inoculating substrate 23 required for both digesters 20, 21, the retention time in the fast digester 21 can be reduced. This allows for increasing the overall throughput of digestion substrate through the biogas plant.

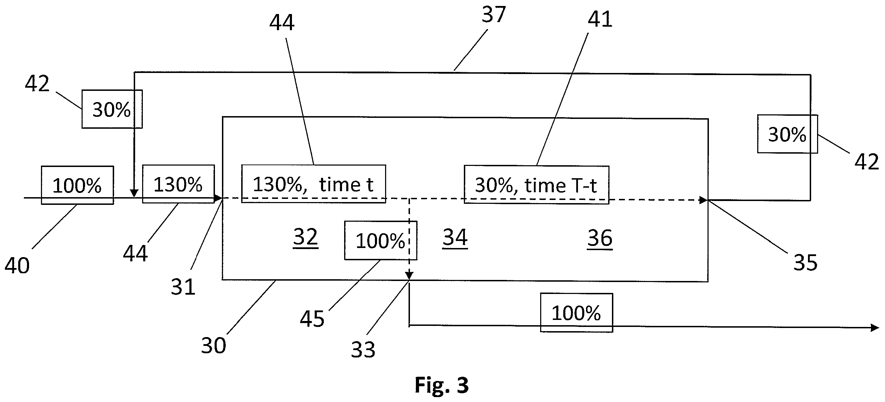

The anaerobic biogas plant shown in Fig. 3 is operated in accordance with the present invention and includes a digester 30 with an inlet 31 in an entry section 32 at one end, a main outlet 33 in a middle section 34, an additional outlet 35 in a rear section 36 at an opposite end and a return line 37 connecting the additional outlet 35 with the inlet 31 for returning some digested substrate back into the digester 30 as self-inoculation substrate. Via the inlet 31 in the entry section 32, the digester 30 is supplied with fresh digestion substrate 40, which is transported in the plug-flow mode through the middle section 34 and towards the rear section 36 of the digester 30. A minor fraction 41 of digestion substrate is digested in the digester 30 for an inoculum retention time T. Then said minor fraction 41 of digestion substrate is removed via the additional outlet 35 and is returned back into the digester 30 to act as inoculation substrate 42. The inoculation substrate 42 is mixed with fresh digestion substrate 40 to obtain a combined digestion substrate 44. The combined digestion substrate 44 is subsequently digested upon passing the digester 30 in the plug-flow mode. A major fraction 45 of combined digestion substrate 44 is not transported to the rear section 36 of the digester 30 but is removed after a shortened retention time t via the main outlet 33 from the middle section 34 of the digester 30. Only the minor fraction 41 of the combined digestion substrate 44 is passed on and retrieved from the rear section 36 of the digester 30 via the additional outlet 35 - again, as described above, to be returned back into the digester 30 and act as inoculation substrate 42.

Compared to known anaerobic biogas plants having a single digester as presented in Fig. 1, the anaerobic biogas plant in accordance with the present invention allows for a significant reduction of the average retention time (i.e. dwell time) of the combined digestion substrate in the digester and thus for increasing the overall throughput of fresh digestion substrate. This will be explained further with a specific example, with process parameters as indicated in Table 1 below:

**Table 1**

| | **Digester A,** **Figure 1** | **Digester B,** **Figure 2** | **Digester C,** **Figure 3** |
|---|---|---|---|
| **Fresh Input** | 100 t/day | 30 t/day + 100 t/day = 130 t/day | 100 t/day |
| **Digester capacity** | 130 t/day for 21 days = 2'730 t | 39 t/day for 21 days + 130 t/day for 7 days = 1'729 t | 130 t/day for 7 days + 30 t/day for 14 days = 1'330 t |
| **Output** | 100 t/day | 130 t/day | 100 t/day |
| **Average retention time** | 2'730 t / 100 t/day = 27 days | 1'729 t / 130 t/day = 13 days | 1'300 t / 100 t/day = 13 days |

Assuming that in a biogas plant according to the prior art as shown in Fig. 1, a total input of 130 tons/day of combined digestion substrate 17 is fed into the digester 10 and digested for a retention time T = 21 days. Said total input 17 consists of 100% fresh digestion substrate 14 and 30% inoculation substrate 16. After the retention time T = 21 days, a major fraction 18 of 100% = 100 tons/day of the digested substrate 15 can be retrieved from the anaerobic biogas plant and a minor fraction 16 of 30% = 30 tons/day is returned to the digestion chamber to act as inoculation substrate. This results in a digester capacity of 2'730 tons. For the combined digestion substrate - including the re-circulated inoculation substrate - this results in an average retention time of 27 days.

In the biogas plant of the prior art shown in Fig. 2, 30 tons/day of fresh digestion substrate 22 and 9 tons/day of a self-inoculation substrate is fed into the inoculation digester 20 and digested therein for 21 days. The 39 tons/day of digested substrate 23 retrieved from the inoculation digester 20 are divided into two fractions: A minor fraction 24 of 9 tons/day is returned back into the inoculation digester 20 and a major fraction 25 of 30 tons/day is introduced into the fast digester 21, where the latter is mixed with 100 tons/day of fresh digestion substrate 22. The fast digester 21 of Fig. 2 is thus fed with a total input of 130 tons/day and said input is digested for a shortened retention time of 7 days. For all digestion substrate together in both digesters, this results in an average retention time of 13 days.

In accordance with the present invention as shown in Fig. 3, the digester 30 of an anaerobic biogas plant is supplied with a total input of 130 tons/day of combined digestion substrate 44, which consists of 100 tons/day of fresh digestion substrate 40 and of 30 tons/day of inoculation substrate 42. A major fraction 45 of 100% = 100 tons/day of the combined digestion substrate 44 is digested for a shortened retention time t of 7 days before being removed through the main outlet 33. A minor fraction 41 of 30% = 30 tons/day of the combined digestion substrate 44 is digested for another 14 days, i.e. for a total prolonged retention time T of 21 days, before being removed via the additional outlet 35 and recirculated into the digester 30 as inoculation substrate 42. For all digestion substrate, i.e. the fresh digestion substrate 40 and the inoculation substrate 42 together, this results in an average retention time of 13 days.

Compared to the anaerobic biogas plant shown in Fig. 1 that requires an average retention time of 27 days, the biogas plant of the present invention has an average retention time of only 13 days (for the same amount of input material). The biogas plants shown in Fig. 2 and 3 have the same average retention time.

Thus, assuming that all digesters have the same digester capacity/size, the overall throughput of the anaerobic biogas plants shown in Fig. 2 and 3 is substantially increased compared to the one shown in Fig. 1.

Although the biogas plant shown in Fig. 2 and the inventive biogas plant shown in Fig. 3 have the same average retention time, the biogas plant of the present invention has the benefit that it requires only one digester, which reduces the building and maintenance costs.

## Claims

1. Anaerobic biogas plant comprising a digester (30) for the digestion of fresh organic digestion substrate (40), said digester (30) having
an entry section (32) with a main inlet (31) at a first end,
a rear section (36) at a second opposite end,
a middle section (34) arranged between the entry section (32) and the rear section (36), and
a stirring unit comprising a shaft extending in the direction from the first end to the second end and having at least one agitating arm for mixing the digestion substrate (44) within the digester (30),
**wherein** the middle section (34) of the digester (30) includes a main outlet (33) for removal of a major fraction (45) of digestion substrate after a shortened retention time t,
the rear section (36) includes an additional outlet (35) for removal of a minor fraction (41) of digestion substrate after an inoculum retention time T, and
the digester (30) includes a separate return line (37) leading from the additional outlet (35) back to the entry section (32) of the digester (30) for allowing a direct return of the minor fraction (41) of digestion substrate removed from the rear section (36) back into the entry section (32) of the digester (30).

2. Anaerobic biogas plant according to Claim 1, wherein the digester (30) is an elongated, horizontal tank, preferably with an essentially constant diameter.

3. Anaerobic biogas plant according to one of Claims 1 or 2, wherein the stirring unit extends from the entry section (32) through the middle section (34) into the rear section (36) of the digester (30).

4. Anaerobic biogas plant according to one of Claims 1 to 3, wherein the shaft of the stirring unit has a multitude of agitating arms, which are provided with blades at their free ends.

5. Anaerobic biogas plant according to one of Claims 1 to 4, wherein the separate return line (37) exclusively connects the rear section (36) with the entry section (32) .

6. Anaerobic biogas plant including two or more digesters (30), all of which being of the type as defined in Claims 1 to 5.

7. Method of digestion in an anaerobic biogas plant having at least one digester (30),
said digester (30) having
an entry section (32) with a main inlet (31) at a first end,
a rear section (36) at a second, opposite end,
a middle section (34) arranged between the entry section (32) and the rear section (36),
a main outlet (33) from the middle section (34), an additional outlet (35) from the rear section (36),
a separate return line (37) leading from the additional outlet (35) directly back to the entry section (32) of the digester (30), and
a stirring unit comprising a shaft extending in the direction from the first end to the second end and having at least one agitating arm for mixing the digestion substrate (44) within the digester (30);
the method comprising the steps of:
a) supplying fresh digestion substrate (40) through the main inlet (31) into the digester (30);
b) removing a minor fraction (41) of digestion substrate via the additional outlet (35) from the rear section (36) of the digester (30) after an inoculum retention time T,
c) returning the minor fraction (41) of digestion substrate via the return line (37) directly back into the entry section (32) of the digester (30) to act as inoculation substrate (42),
d) mixing the inoculation substrate (42) with fresh digestion substrate (40) to provide a combined digestion substrate (44), and
e) removing a major fraction (45) of combined digestion substrate (44) via the main outlet (33) from the middle section (34) of the digester (30) after a shortened retention time t,
wherein the major fraction (45) of combined digestion substrate (44) is larger than the minor fraction (41) of combined digestion substrate (44) that is returned as inoculation substrate (42), and the shortened retention time t is shorter than the inoculum retention time T.

8. Digestion method according to Claim 7, wherein the minor fraction (41) of digestion substrate removed from the rear section (36) is directly returned back into the entry section (32) of the digester (30) without further processing.

9. Digestion method according to Claim 7 or 8, wherein the minor fraction (41) of digestion substrate removed from the rear section (36) is less than 50%, preferably less than 40%, more preferably about 30% or less, of the amount of fresh digestion substrate (40) fed into the digester.

10. Digestion method according to one of Claims 7 to 9, wherein in step e), the major fraction (45) of combined digestion substrate (44) is removed after a shortened retention time t of less than 50%, preferably less than 40%, more preferably about a third of the inoculum retention time T.

11. Digestion method according to one of Claims 7 to 10, wherein in step e), the major fraction (45) of combined digestion substrate (44) is removed after a shortened retention time t of at least 2 days, more preferably at least 4 days and less than 10 days, most preferably between 2 and 6 days.

12. Digestion method according to one of Claims 7 to 11, wherein in step b), the minor fraction (41) of digestion substrate is removed after an inoculum retention time T of at least 7 days, more preferably at least 14 days, most preferably about 21 days.

13. Digestion method according to one of Claims 7 to 12, wherein the removal and return of the minor fraction (41) of digestion substrate (44) from and back into the digester (30) occurs in a continuous fashion or in regular time intervals.

14. Digestion method according to one of Claims 7 to 12, wherein the removal and return of the minor fraction (41) of digestion substrate (44) from and back into the digester (30) occurs in dependence of at least one condition parameter measured within the digester, said at least one condition parameter is selected from the group consisting of pH, volume of substrate, temperature, content of volatile fatty acids, free ammonia concentration and partial gas pressure.

15. Digestion method according to Claim 7, wherein the digester (30) is operated in the plug-flow mode.

## Patentansprüche

1. Anaerobe Biogasanlage mit einem Fermenter (30) für die Vergärung von frischem organischem Gärsubstrat (40), wobei der Fermenter (30) Folgendes aufweist:
einen Eingangsabschnitt (32) mit einem Haupteinlass (31) an einem ersten Ende,
einen hinteren Abschnitt (36) an einem zweiten, gegenüberliegenden Ende,
einen mittleren Abschnitt (34), der zwischen dem Eingangsabschnitt (32) und dem hinteren Abschnitt (36) angeordnet ist, und
eine Rühreinheit, die eine Welle umfasst, die sich in der Richtung vom ersten Ende zum zweiten Ende erstreckt und mindestens einen Rührarm zum Mischen des Gärsubstrats (44) innerhalb des Fermenters (30) aufweist,
wobei der mittlere Abschnitt (34) des Fermenters (30) einen Hauptauslass (33) zum Entfernen einer Hauptfraktion (45) des Gärsubstrats nach einer verkürzten Verweilzeit t aufweist,
der hintere Abschnitt (36) einen zusätzlichen Auslass (35) zur Entnahme einer kleineren Fraktion (41) des Gärsubstrats nach einer Inokulum-Verweilzeit T aufweist, und
der Fermenter (30) eine separate Rückführleitung (37) aufweist, die vom zusätzlichen Auslass (35) zurück zum Eingangsabschnitt (32) des Fermenters (30) führt, um eine direkte Rückführung der kleineren Fraktion (41) des aus dem hinteren Abschnitt (36) entnommenen Gärsubstrats zurück in den Eingangsabschnitt (32) des Fermenters (30) zu ermöglichen.

2. Anaerobe Biogasanlage nach Anspruch 1, wobei der Fermenter (30) ein länglicher, horizontaler Behälter ist, vorzugsweise mit einem im Wesentlichen konstanten Durchmesser.

3. Anaerobe Biogasanlage nach einem der Ansprüche 1 oder 2, wobei sich das Rührwerk vom Eingangsabschnitt (32) durch den mittleren Abschnitt (34) in den hinteren Abschnitt (36) des Fermenters (30) erstreckt.

4. Anaerobe Biogasanlage nach einem der Ansprüche 1 bis 3, wobei die Welle des Rührwerks eine Vielzahl von Rührarmen aufweist, die an ihren freien Enden mit Schaufeln versehen sind.

5. Anaerobe Biogasanlage nach einem der Ansprüche 1 bis 4, wobei die separate Rückführleitung (37) ausschließlich den hinteren Abschnitt (36) mit dem Eingangsabschnitt (32) verbindet.

6. Anaerobe Biogasanlage mit zwei oder mehr Fermentern (30), die alle nach einem der Ansprüche 1 bis 5 ausgebildet sind.

7. Verfahren zur Vergärung in einer anaeroben Biogasanlage mit mindestens einem Fermenter (30),
wobei der Fermenter (30) Folgendes aufweist:
einen Eingangsabschnitt (32) mit einem Haupteinlass (31) an einem ersten Ende,
einen hinteren Abschnitt (36) an einem zweiten, gegenüberliegenden Ende,
einen mittleren Abschnitt (34), der zwischen dem Eingangsabschnitt (32) und dem hinteren Abschnitt (36) angeordnet ist,
einen Hauptauslass (33) aus dem mittleren Abschnitt (34),
einen zusätzlichen Auslass (35) aus dem hinteren Abschnitt (36),
eine separate Rückführleitung (37), die vom zusätzlichen Auslass (35) direkt zurück zum Eingangsabschnitt (32) des Fermenters (30) führt, und eine Rühreinheit mit einer Welle, die sich in der Richtung vom ersten Ende zum zweiten Ende erstreckt und mindestens einen Rührarm zum Mischen des Gärsubstrats (44) innerhalb des Fermenters (30) aufweist;
wobei das Verfahren die folgenden Schritte umfasst:
a) Zuführen von frischem Gärsubstrat (40) durch den Haupteinlass (31) in den Fermenter (30);
b) Entnahme einer kleineren Fraktion (41) des Gärsubstrats über den zusätzlichen Auslass (35) aus dem hinteren Abschnitt (36) des Fermenters (30) nach einer Inokulum-Verweilzeit T,
c) Rückführung der kleinen Fraktion (41) des Gärsubstrats über die Rückführleitung (37) direkt zurück in den Eingangsabschnitt (32) des Fermenters (30), um als Impfsubstrat (42) zu dienen,
d) Mischen des Impfsubstrats (42) mit frischem Gärsubstrat (40), um ein kombiniertes Gärsubstrat (44) zu erhalten, und
e) Entfernen einer Hauptfraktion (45) des kombinierten Gärsubstrats (44) über den Hauptauslass (33) aus dem mittleren Abschnitt (34) des Fermenters (30) nach einer verkürzten Verweilzeit t,
wobei die Hauptfraktion (45) des kombinierten Gärsubstrats (44) grösser ist als die als Impfsubstrat (42) zurückgeführte kleinere Fraktion (41) des kombinierten Gärsubstrats (44), und die verkürzte Verweilzeit t kürzer ist als die Inokulum-Verweilzeit T.

8. Fermentationsverfahren nach Anspruch 7, wobei die aus dem hinteren Abschnitt (36) entnommene kleinere Fraktion (41) des Gärsubstrats ohne weitere Bearbeitung direkt in den Eingangsabschnitt (32) des Fermenters (30) zurückgeführt wird.

9. Fermentationsverfahren nach Anspruch 7 oder 8, wobei die kleinere Fraktion (41) des aus dem hinteren Abschnitt (36) entfernten Gärsubstrats weniger als 50%, vorzugsweise weniger als 40%, besonders bevorzugt etwa 30% oder weniger, der Menge des in den Fermenter eingebrachten frischen Gärsubstrats (40) beträgt.

10. Fermentationsverfahren nach einem der Ansprüche 7 bis 9, wobei in Schritt e) die Hauptfraktion (45) des kombinierten Gärsubstrats (44) nach einer verkürzten Verweilzeit t von weniger als 50%, vorzugsweise weniger als 40%, besonders bevorzugt etwa einem Drittel der Inokulum-Verweilzeit T entfernt wird.

11. Fermentationsverfahren nach einem der Ansprüche 7 bis 10, wobei in Schritt e) die Hauptfraktion (45) des kombinierten Gärsubstrats (44) nach einer verkürzten Verweilzeit t von mindestens 2 Tagen, vorzugsweise mindestens 4 Tagen und weniger als 10 Tagen, besonders bevorzugt zwischen 2 und 6 Tagen, entfernt wird.

12. Fermentationsverfahren nach einem der Ansprüche 7 bis 11, wobei in Schritt b) die kleinere Fraktion (41) des Gärsubstrats nach einer Inokulum-Verweilzeit T von mindestens 7 Tagen, bevorzugter von mindestens 14 Tagen, am meisten bevorzugt von etwa 21 Tagen entfernt wird.

13. Fermentationsverfahren nach einem der Ansprüche 7 bis 12, wobei die Entnahme und Rückführung der kleineren Fraktion (41) des Gärsubstrats (44) aus dem und zurück in den Fermenter (30) kontinuierlich oder in regelmässigen Zeitabständen erfolgt.

14. Fermentationsverfahren nach einem der Ansprüche 7 bis 12, wobei die Entnahme und Rückführung der kleineren Fraktion (41) des Gärsubstrats (44) aus dem und zurück in den Fermenter (30) in Abhängigkeit von mindestens einem im Fermenter gemessenen Zustandsparameter erfolgt, wobei der mindestens eine Zustandsparameter ausgewählt ist aus der Gruppe bestehend aus pH-Wert, Substratvolumen, Temperatur, Gehalt an flüchtigen Fettsäuren, Konzentration an freiem Ammoniak und Partialgasdruck.

15. Fermentationsverfahren nach Anspruch 7, wobei der Fermenter (30) im Pfropfenstrombetrieb betrieben wird.

## Revendications

1. Installation de biogaz anaérobie comprenant un digesteur (30) pour la digestion d'un substrat de digestion organique frais (40), ledit digesteur (30) ayant
une section d'entrée (32) avec une entrée principale (31) à une première extrémité,
une section arrière (36) à une deuxième extrémité opposée,
une section médiane (34) disposée entre la section d'entrée (32) et la section arrière (36), et
une unité d'agitation comprenant un arbre s'étendant dans la direction de la première extrémité à la seconde extrémité et ayant au moins un bras d'agitation pour mélanger le substrat de digestion (44) à l'intérieur du digesteur (30),
dans lequel la section médiane (34) du digesteur (30) comprend une sortie principale (33) pour l'élimination d'une fraction principale (45) du substrat de digestion après un temps de rétention raccourci t,
la section arrière (36) comprend une sortie supplémentaire (35) pour l'élimination d'une fraction mineure (41) du substrat de digestion après un temps de rétention de l'inoculum T, et
le digesteur (30) comprend une conduite de retour séparée (37) menant de la sortie supplémentaire (35) vers la section d'entrée (32) du digesteur (30) pour permettre un retour direct de la fraction mineure (41) du substrat de digestion retiré de la section arrière (36) vers la section d'entrée (32) du digesteur (30).

2. Installation de biogaz anaérobie selon la revendication 1, dans laquelle le digesteur (30) est une cuve allongée, horizontale, de préférence avec un diamètre essentiellement constant.

3. Installation de biogaz anaérobie selon l'une des revendications 1 ou 2, dans laquelle l'unité d'agitation s'étend de la section d'entrée (32) à la section arrière (36) du digesteur (30) en passant par la section médiane (34).

4. Installation de biogaz anaérobie selon l'une des revendications 1 à 3, dans laquelle l'arbre de l'unité d'agitation présente une multitude de bras d'agitation, qui sont munis de pales à leurs extrémités libres.

5. Installation de biogaz anaérobie selon l'une des revendications 1 à 4, dans laquelle la conduite de retour séparée (37) relie exclusivement la section arrière (36) à la section d'entrée (32).

6. Installation de biogaz anaérobie comprenant deux ou plusieurs digesteurs (30), tous étant du type défini dans les revendications 1 à 5.

7. Procédé de digestion dans une installation de biogaz anaérobie comprenant au moins un digesteur (30), ledit digesteur (30) ayant
une section d'entrée (32) avec une entrée principale (31) à une première extrémité,
une section arrière (36) à une deuxième extrémité opposée,
une section médiane (34) disposée entre la section d'entrée (32) et la section arrière (36),
une sortie principale (33) de la section médiane (34), une sortie supplémentaire (35) de la section arrière (36),
une conduite de retour séparée (37) menant de la sortie supplémentaire (35) directement à la section d'entrée (32) du digesteur (30), et
une unité d'agitation comprenant un arbre s'étendant dans la direction de la première extrémité à la seconde extrémité et ayant au moins un bras d'agitation pour mélanger le substrat de digestion (44) à l'intérieur du digesteur (30);
le procédé comprenant les étapes consistant à :
a) fournir un substrat de digestion frais (40) à travers l'entrée principale (31) dans le digesteur (30) ;
b) retirer une fraction mineure (41) du substrat de digestion via la sortie supplémentaire (35) de la section arrière (36) du digesteur (30) après un temps de rétention de l'inoculum T,
c) le retour de la fraction mineure (41) du substrat de digestion via la conduite de retour (37) directement dans la section d'entrée (32) du digesteur (30) pour agir comme substrat d'inoculation (42),
d) mélanger le substrat d'inoculation (42) avec le substrat de digestion frais (40) pour fournir un substrat de digestion combiné (44), et
e) retirer une fraction majeure (45) du substrat de digestion combiné (44) via la sortie principale (33) de la section centrale (34) du digesteur (30) après un temps de rétention raccourci t,
dans lequel la fraction majeure (45) du substrat de digestion combinée (44) est plus grande que la fraction mineure (41) du substrat de digestion combinée (44) qui est retournée comme substrat d'inoculation (42), et le temps de rétention raccourci t est plus court que le temps de rétention de l'inoculum T.

8. Procédé de digestion selon la revendication 7, dans lequel la fraction mineure (41) de substrat de digestion retirée de la section arrière (36) est directement retournée dans la section d'entrée (32) du digesteur (30) sans autre traitement.

9. Procédé de digestion selon la revendication 7 ou 8, dans lequel la fraction mineure (41) de substrat de digestion retirée de la section arrière (36) est inférieure à 50%, de préférence inférieure à 40%, plus préférablement d'environ 30% ou moins, de la quantité de substrat de digestion frais (40) introduite dans le digesteur.

10. Procédé de digestion selon l'une des revendications 7 à 9, dans lequel, à l'étape e), la fraction majeure (45) de substrat de digestion combiné (44) est retirée après un temps de rétention t raccourci de moins de 50%, de préférence moins de 40%, plus préférablement d'environ un tiers du temps de rétention T de l'inoculum.

11. Procédé de digestion selon l'une des revendications 7 à 10, dans lequel à l'étape e), la fraction majeure (45) du substrat de digestion combiné (44) est éliminée après un temps de rétention raccourci t d'au moins 2 jours, plus préférentiellement d'au moins 4 jours et inférieur à 10 jours, plus préférentiellement entre 2 et 6 jours.

12. Procédé de digestion selon l'une des revendications 7 à 11, dans lequel à l'étape b), la fraction mineure (41) de substrat de digestion est éliminée après un temps de rétention T de l'inoculum d'au moins 7 jours, plus préférentiellement d'au moins 14 jours, tout particulièrement d'environ 21 jours.

13. Procédé de digestion selon l'une des revendications 7 à 12, dans lequel l'élimination et le retour de la fraction mineure (41) du substrat de digestion (44) depuis et vers le digesteur (30) se produisent de manière continue ou à intervalles de temps réguliers.

14. Procédé de digestion selon l'une des revendications 7 à 12, dans lequel l'élimination et le retour de la fraction mineure (41) du substrat de digestion (44) depuis et vers le digesteur (30) se produisent en fonction d'au moins un paramètre de condition mesuré à l'intérieur du digesteur, ledit au moins un paramètre de condition est choisi dans le groupe constitué par le pH, le volume de substrat, la température, la teneur en acides gras volatils, la concentration en ammoniac libre et la pression partielle de gaz.

15. Procédé de digestion selon la revendication 7, dans lequel le digesteur (30) fonctionne en mode d'écoulement piston.
